# EUROPEAN PATENT APPLICATION

(11) **EP 4 442 819 A1**
(43) Date of publication of application: **09.10.2024**
(21) Application number: 22899856.3
(22) Date of filing: 22.06.2022
(51) Int. Cl.: C12N 9/12

(54) **TAQ ENZYME MUTANT, PREPARATION METHOD, AND APPLICATION THEREOF**

(30) Priority: 30.11.2021 CN 202111445100
(71) Applicant: Daan Gene Co., Ltd., Guangzhou, Guangdong 510665 (CN)
(72) Inventor: JIANG, Xiwen, Guangzhou, Guangdong 510665 (CN); ZHANG, Wei, Guangzhou, Guangdong 510665 (CN); LIAN, Xianlan, Guangzhou, Guangdong 510665 (CN); LU, Xuelan, Guangzhou, Guangdong 510665 (CN)
(74) Representative: Viering, Jentschura & Partner mbB Patent- und Rechtsanwälte
(86) International application number: PCT/CN2022/100276
(87) International publication number: WO 2023/098036

(57) **Abstract**

The present application discloses mutants of Taq enzyme, a method of preparation thereof and an application thereof. In the present application, the mutants of Taq enzyme comprises: an amino acid sequence having at least 70% identity to the amino acid sequence shown in SEQ ID NO:2; amino acid sequence is mutated at one or more sites selected from the group consisting of: D335, G499, E634, F769 and a combination thereof. The mutants of Taq enzyme provided in the present application have high amplification activity, yield more amplification products compared to wild-type Taq enzyme at the same number of PCR cycles, are both whole blood and high salt resistant, and do not lose 5'~3' exonuclease activity.

## Description

### Cross-reference to related application

This patent application claims priority for the Chinese patent application filed on November 30, 2021, with application number 202111445100.1 and invention name "TAQ ENZYME MUTANT, PREPARATION METHOD, AND APPLICATION THEREOF", the full text of which is incorporated herein by reference.

### Technical Field

The present invention relates to the field of PCR detection, in particular to mutants of Taq enzyme and methods of preparation and use thereof.

### Background

Taq enzyme is a heat-resistant DNA polymerase derived from the heat-resistant bacterium Thermus aquaticus, with a molecular weight of 94 KDa. In the presence of magnesium ions, its optimal reaction temperature is 75-80°C, and its active half-life is 40 minutes at 95°C. It has 5'-3' nucleic acid exonuclease activity. Because of its high temperature resistance, Taq enzyme is widely used in polymerase chain reaction (PCR). Taq enzyme is the first choice for nucleic acid amplification, detection and other reactions. Commercial Taq enzymes are cloned and expressed using the E. coli prokaryotic expression system. Modern molecular biological detection technology requires increasing sensitivity, precision, and durability of the PCR reaction, and wild-type Taq enzyme cannot fully meet the needs of practical applications.

In order to make them more adaptable for use in specific technologies, various forms of mutational modification of Taq enzyme sequences have been carried out in the prior art, such as, for example, (1) the addition of structural domains; (2) the removal of non-essential structural domains on Taq enzyme; (3) the mixing of polymerases with other polymerases having 3'~5' exonuclease activity; and (4) site-specific mutagenesis.

Wherein site-specific mutagenesis refers to site-specific mutagenesis of amino acids in the active site, magnesium ion binding site, and DNA binding site on Taq enzyme in order to improve the affinity of each site for substrate, template, and primer, and thereby improve the tolerance to various inhibitors.

However, the inventors found in their research that although site-specific mutagenesis of Taq enzyme sequences can improve DNA polymerization activity to a certain extent (*Mutant Taq DNA polymerases with improved elongation ability as a useful reagent for genetic engineering. Front Microbiol 5:461. doi: 10.3389*/*fmicb.2014.00461*), but it results in a decrease in 5'~3' exonuclease activity and the mutant Taq enzyme is not resistant to whole blood and high salt, which is unfavorable for clinical detection.

Therefore, it is particularly important to develop a Taq enzyme mutant that does not lose 5'-3' exonuclease activity and is resistant to whole blood and high salt.

### Summary of invention

It is an object of the present invention to provide a mutant of Taq enzyme.

It is another object of the present invention to provide a nucleic acid molecule encoding the above mutants of Taq enzyme.

It is another object of the present invention to provide a vector.

It is another object of the present invention to provide a host cell.

It is another object of the present invention to provide a method of producing each of the mutants of Taq enzyme.

It is another object of the present invention to provide a kit containing a mutant of Taq enzyme.

In order to solve the above technical problem, a first aspect of the present invention provides mutants of Taq enzyme, wherein the mutants of Taq enzyme comprising: an amino acid sequence having at least 70% identity to the amino acid sequence shown in SEQ ID NO:2, wherein the amino acid sequence being mutated at one or more sites selected from the group consisting of: D335, G499, E634, F769, or a combination thereof.

In some preferred embodiments, wherein the amino acid sequence is mutated at any one or more sites selected from the group consisting of D335V, G499K, E634G and F769I.

In some preferred embodiments, wherein the amino acid sequence is mutated at four sites in the following group: D335V, G499K, E634G and F769I.

In some preferred embodiments, wherein the mutant of Taq enzyme has a resistance to sodium chloride of not less than 70 mM, more preferably not less than 80 mM, more preferably not less than 90 mM, more preferably not less than 100 mM, more preferably not less than 130 mM, more preferably not less than 150 mM, and most preferably not less than 180 mM.

In some preferred embodiments, wherein the mutant of Taq enzyme is resistant to potassium chloride not less than 100 mM, more preferably not less than 110 mM, more preferably not less than 120 mM, more preferably not less than 130 mM, more preferably not less than 140 mM, more preferably not less than 150 mM, more preferably not less than 180 mM, more preferably not less than 190 mM, most preferably not less than 200 mM.

In some preferred embodiments, wherein the mutant of Taq enzyme is resistant to EDTA whole blood by not less than 5% (percentage herein refers to plasma volume as a percentage of total PCR solution system volume), more preferably not less than 7%, more preferably not less than 10%, more preferably not less than 20%, more preferably not less than 30%, more preferably not less than 40%, most preferably not less than45%.

In some preferred embodiments, wherein the mutant of Taq enzyme is resistant to heparin whole blood by not less than 1% (percentage herein refers to plasma volume as a percentage of total PCR solution system volume), more preferably not less than 3%, more preferably not less than 5%, more preferably not less than 10%, more preferably not less than 20%, preferably not less than 30%, and most preferably not less than 35%.

SEQ ID NO: 1 (wild-type Taq enzyme DNA as follows):

ATGCGTGGCATGCTGCCTCTGTTTGAACCGAAAGGCCGCGTGTTACT
GGTTGATGGCCATCATCTGGCCTATCGTACCTTTCACGCACTGAAGGGTC
TGACCACAAGCCGTGGCGAGCCGGTTCAGGCCGTGTACGGCTTCGCCAAA
AGCCTGCTGAAAGCCCTGAAAGAAGACGGCGATGCCGTGATCGTGGTTTT
CGACGCCAAGGCACCGAGCTTTCGTCATGAGGCTTATGGTGGCTATAAAG
CCGGTCGCGCACCGACACCGGAGGATTTCCCGCGCCAGCTGGCCTTAATT
AAAGAGCTGGTGGATCTGTTAGGCCTGGCCCGCCTGGAGGTTCCGGGTTA
TGAAGCCGACGACGTGCTGGCCAGCTTAGCCAAGAAGGCAGAGAAAGAG
GGCTATGAGGTGCGCATTCTGACCGCAGACAAGGACCTGTATCAGCTGCT

GAGCGATCGCATTCATGTGCTGCATCCGGAGGGCTACCTGATTACCCCGG
CCTGGCTGTGGGAAAAATATGGTCTGCGCCCGGATCAATGGGCAGATTAT
CGCGCACTGACAGGCGATGAAAGCGATAACCTGCCGGGTGTTAAAGGCA
TTGGCGAGAAGACAGCACGTAAGCTGCTGGAAGAATgGGGTAgCCTGGAA
GCCCTGTTAAAGAACCTGGACCGTCTGAAGCCGGCAATCCGCGAGAAGA
TTCTGGCCCACATGGATGATCTGAAGCTGAGTTGGGATCTGGCCAAAGTT
CGCACAGACTTACCGCTGGAAGTGGACTTTGCCAAGCGTCGTGAACCGGA
CCGCGAACGTCTGCGTGCCTTCTTAGAACGCCTGGAATTCGGTAGCCTGC
TGCACGAATTTGGTCTGCTGGAAAGCCCGAAAGCCTTAGAAGAAGCTCCG
TGGCCGCCTCCGGAAGGTGCCTTTGTTGGCTTTGTTCTGAGCCGCAAAGA
GCCGATGTGGGCAGATTTACTGGCCCTGGCCGCAGCACGCGGTGGTCGTG
TTCATCGTGCCCCGGAACCGTATAAAGCCCTGCGCGACCTGAAAGAGGCA
CGTGGTCTGTTAGCAAAGGACCTGAGCGTGTTAGCACTGCGTGAAGGTCT
GGGCTTACCGCCTGGTGATGATCCGATGCTGCTGGCCTACCTGCTGGACC
CGAGCAACACCACCCCTGAGGGTGTGGCACGCCGTTATGGTGGTGAATGG
ACCGAGGAAGCAGGTGAACGCGCCGCACTGAGTGAGCGTCTGTTTGCAA
ACCTGTGGGGCCGTCTGGAAGGCGAGGAACGTCTGCTGTGGCTGTACCGC
GAAGTGGAACGCCCGCTGAGCGCCGTTTTAGCCCACATGGAGGCCACCG
GCGTTCGCTTAGATGTGGCCTATCTGCGCGCCCTGAGCCTGGAAGTTGCA
GAAGAAATCGCCCGTCTGGAGGCAGAGGTGTTTCGTTTAGCCGGCCATCC
GTTCAACCTGAATAGCCGCGACCAGCTGGAACGCGTGCTGTTTGATGAAC
TGGGCTTACCGGCCATTGGCAAGACCGAGAAGACCGGTAAACGTAGTAC
CAGTGCAGCCGTTCTGGAAGCACTGCGTGAAGCCCACCCTATTGTGGAAA
AGATTCTGCAATATCGCGAGTTAACCAAACTGAAGAGCACCTACATCGAT
CCTCTGCCTGATCTGATTCACCCGCGCACCGGTCGTCTGCACACCCGCTTT
AATCAAACAGCCACAGCCACCGGTCGTCTGAGCAGCAGCGATCCGAACTT
ACAGAACATTCCGGTGCGCACCCCTCTGGGCCAACGCATTCGTCGCGCCT
TCATTGCCGAAGAAGGCTGGTTACTGGTGGCCCTGGACTACAGCCAGATT
GAGCTGCGTGTTTTAGCCCATCTGAGCGGCGACGAAAACCTGATTCGCGT
GTTTCAAGAGGGCCGCGATATTCATACAGAGACCGCCAGCTGGATGTTTG
GTGTTCCTCGCGAAGCCGTTGATCCGCTGATGCGCCGCGCAGCCAAAACA
ATTAACTTCGGTGTGCTGTATGGTATGAGCGCCCACCGTTTAAGCCAGGA
GCTGGCCATTCCGTATGAAGAGGCCCAGGCATTTATCGAGCGCTACTTCC
AGAGTTTCCCTAAGGTGCGTGCATGGATCGAGAAAACACTGGAGGAAGG
TCGCCGTCGTGGCTACGTGGAAACCCTGTTTGGCCGCCGTCGCTATGTGC
CGGATCTGGAAGCCCGCGTGAAAAGCGTTCGTGAGGCAGCAGAGCGCAT
GGCATTTAATATGCCGGTGCAGGGTACCGCAGCCGACCTGATGAAACTGG
CAATGGTGAAGCTGTTTCCGCGTCTGGAAGAGATGGGCGCCCGCATGTTA
CTGCAGGTGCACGACGAACTGGTTCTGGAGGCACCGAAAGAACGTGCCG
AAGCAGTGGCACGCCTGGCCAAAGAGGTTATGGAAGGCGTTTATCCGTTA
GCCGTTCCGCTGGAGGTGGAGGTTGGCATTGGCGAAGACTGGCTGAGTGC
AAAAGAATAA

SEQ ID NO:2 (the amino acid sequence of wild-type Taq enzyme is as follows)

In some preferred embodiments, wherein the mutant of Taq enzyme comprise an amino acid sequence having at least 80% identity with the amino acid sequence shown in SEQ ID NO:1; more preferably, the mutant of Taq enzyme comprises an amino acid sequence having at least 90% identity with the amino acid sequence shown in SEQ ID NO:1; more preferably, the mutant of Taq enzyme comprises an amino acid sequence having at least 95% identity with the amino acid sequence shown in SEQ ID NO:1.

A second aspect of the present invention further provides a nucleotide molecule, wherein the nucleotide molecule encoding a mutant of Taq enzyme described in the first aspect of the present invention.

A third aspect of the present invention further provides a vector comprising the nucleic acid molecule described in the second aspect of the present invention.

A fourth aspect of the present invention further provides a host cell, wherein the host cell contains a nucleotide molecule described in the second aspect of the present invention, or the chromosome integrates a nucleotide molecule described in the second aspect of the present invention.

In some preferred embodiments, wherein the host cell is a prokaryotic cell, or a eukaryotic cell.

In some preferred embodiments, wherein the prokaryotic cell is E. coli.

In some preferred embodiments, wherein the eukaryotic cell is a yeast cell.

A fifth aspect of the present invention further provides a kit containing mutants of Taq enzyme described in the first aspect of the present invention.

A sixth aspect of the present invention further provides a method of preparing the mutants of Taq enzyme described in the first aspect of the present invention, wherein the method comprising the steps of.
(i) culturing the host cell described in the fourth aspect of the present invention under suitable conditions so as to express the mutants of Taq enzyme; and
(ii) isolating the mutants of Taq enzyme.

A seventh aspect of the present invention also provides a use for the kit described in the fifth aspect of the present invention which is used for DNA sequencing, DNA labeling, primer extension, amplification, and the like.

The present invention has at least the following advantages over the prior art:
(1) The mutants of Taq enzyme provided by the present invention has high amplification activity, and can obtain more amplification products than the wild-type Taq enzyme at the same number of PCR cycles;
(2) The mutants of Taq enzyme provided by the present invention are resistant to both whole blood and high salt;
(3) The mutants of Taq enzyme provided by the present invention do not lose 5'~3' exonuclease activity.

It should be understood that, within the scope of the present invention, each of the above technical features of the present invention and each of the technical features specifically described below (e.g., in the embodiments) may be combined with each other, thereby constituting a new or preferred technical solution. For the sake of limitation of space, we will not repeat all of them herein.

### Description of drawings

One or more embodiments are exemplarily illustrated by the figures in the accompanying drawings, which do not constitute a limitation of the embodiments.
FIG. 1 is a graph of Taq-aCM protein purification electrophoresis according to embodiments of the present invention;
FIG. 2 is a graph of the results of wild-type Taq enzyme anti-NaCl experiments according to embodiments of the present invention;
FIG. 3 is a graph of the results of Taq-aCM anti-NaCl experiments according to embodiments of the present invention;
FIG. 4 is a graph of the results of the wild-type Taq enzyme anti-KCl experiment according to embodiments of the present invention;
FIG. 5 is a graph of results of Taq-aCM anti-KCl experiments according to embodiments of the present invention;
FIG. 6 is a graph of the results of the wild-type Taq enzyme anti-EDTA blood experiment according to embodiments of the present invention;
FIG. 7 is a graph of the results of the Taq-aCM anti-EDTA blood experiment according to embodiments of the present invention;
FIG. 8 is a graph of the results of the wild-type Taq enzyme anti-heparin blood experiment according to embodiments of the present invention;
FIG. 9 is a graph of Taq-aCM anti-heparin blood experiment results according to embodiments of the present invention;
FIG. 10 is a graph of RFU-cycling curves according to Example 8 of the present invention.

### Embodiments

The wild-type Taq enzyme has difficulty amplifying under some extreme conditions where its polymerization activity is low, such as high salt, whole blood environments. Although attempts have been made in the prior art to mutate wild-type Taq enzyme to enhance its resistance to extreme conditions, but with little success, and it often leads to the decrease of 5'~3' exonuclease activity.

Through extensive and in-depth research, the present inventors have screened out mutants of Taq enzyme with good performance by applying the steps of the site-specific mutagenesis technology. The mutants of Taq enzyme of the present invention have good polymerization activity, good resistance to high salt and whole blood, without loss of 5'~3' exonuclease activity, and are suitable for clinical use.

Some preferred embodiments of the present invention provide the mutants of Taq enzyme, which comprising: an amino acid sequence having at least 70% identity to the amino acid sequence shown in SEQ ID NO:2, wherein the amino acid sequence being mutated at one or more sites selected from the group consisting of: D335, G499, E634, F769, and a combination thereof.

In some preferred embodiments, the amino acid sequence is mutated at any one or more sites selected from the group consisting of:
D335V, G499K, E634G and F769I.

In some preferred embodiments, wherein the amino acid sequence is mutated at four sites in the following group: D335V, G499K, E634G and F769I.

In some preferred embodiments, wherein the mutant of Taq enzyme has a resistance to sodium chloride of not less than 70 mM, more preferably not less than 80 mM, more preferably not less than 90 mM, more preferably not less than 100 mM, more preferably not less than 130 mM, more preferably not less than 150 mM, and most preferably not less than 180 mM.

In some preferred embodiments, wherein the mutant of Taq enzyme is resistant to potassium chloride not less than 100 mM, more preferably not less than 110 mM, more preferably not less than 120 mM, more preferably not less than 130 mM, more preferably not less than 140 mM, more preferably not less than 150 mM, more preferably not less than 180 mM, more preferably not less than 190 mM, most preferably not less than 200 mM.

In some preferred embodiments, wherein the mutant of Taq enzyme is resistant to EDTA whole blood by not less than 5%, more preferably not less than 7%, more preferably not less than 10%, more preferably not less than 20%, more preferably not less than 30%, more preferably not less than 40%, most preferably not less than 45%.

In some preferred embodiments, wherein the mutants] of Taq enzyme is resistant to heparin whole blood by not less than 1%. more preferably not less than 3%, more preferably not less than 5%, more preferably not less than 10%, more preferably not less than 20%, more preferably not less than 30%, and most preferably not less than 35%.

SEQ ID NO: 1 (wild-type Taq enzyme DNA sequence as follows):

ATGCGTGGCATGCTGCCTCTGTTTGAACCGAAAGGCCGCGTGTTACT
GGTTGATGGCCATCATCTGGCCTATCGTACCTTTCACGCACTGAAGGGTC
TGACCACAAGCCGTGGCGAGCCGGTTCAGGCCGTGTACGGCTTCGCCAAA
AGCCTGCTGAAAGCCCTGAAAGAAGACGGCGATGCCGTGATCGTGGTTTT
CGACGCCAAGGCACCGAGCTTTCGTCATGAGGCTTATGGTGGCTATAAAG
CCGGTCGCGCACCGACACCGGAGGATTTCCCGCGCCAGCTGGCCTTAATT
AAAGAGCTGGTGGATCTGTTAGGCCTGGCCCGCCTGGAGGTTCCGGGTTA
TGAAGCCGACGACGTGCTGGCCAGCTTAGCCAAGAAGGCAGAGAAAGAG
GGCTATGAGGTGCGCATTCTGACCGCAGACAAGGACCTGTATCAGCTGCT
GAGCGATCGCATTCATGTGCTGCATCCGGAGGGCTACCTGATTACCCCGG
CCTGGCTGTGGGAAAAATATGGTCTGCGCCCGGATCAATGGGCAGATTAT
CGCGCACTGACAGGCGATGAAAGCGATAACCTGCCGGGTGTTAAAGGCA
TTGGCGAGAAGACAGCACGTAAGCTGCTGGAAGAATgGGGTAgCCTGGAA
GCCCTGTTAAAGAACCTGGACCGTCTGAAGCCGGCAATCCGCGAGAAGA
TTCTGGCCCACATGGATGATCTGAAGCTGAGTTGGGATCTGGCCAAAGTT
CGCACAGACTTACCGCTGGAAGTGGACTTTGCCAAGCGTCGTGAACCGGA
CCGCGAACGTCTGCGTGCCTTCTTAGAACGCCTGGAATTCGGTAGCCTGC
TGCACGAATTTGGTCTGCTGGAAAGCCCGAAAGCCTTAGAAGAAGCTCCG
TGGCCGCCTCCGGAAGGTGCCTTTGTTGGCTTTGTTCTGAGCCGCAAAGA
GCCGATGTGGGCAGATTTACTGGCCCTGGCCGCAGCACGCGGTGGTCGTG
TTCATCGTGCCCCGGAACCGTATAAAGCCCTGCGCGACCTGAAAGAGGCA
CGTGGTCTGTTAGCAAAGGACCTGAGCGTGTTAGCACTGCGTGAAGGTCT
GGGCTTACCGCCTGGTGATGATCCGATGCTGCTGGCCTACCTGCTGGACC
CGAGCAACACCACCCCTGAGGGTGTGGCACGCCGTTATGGTGGTGAATGG
ACCGAGGAAGCAGGTGAACGCGCCGCACTGAGTGAGCGTCTGTTTGCAA
ACCTGTGGGGCCGTCTGGAAGGCGAGGAACGTCTGCTGTGGCTGTACCGC
GAAGTGGAACGCCCGCTGAGCGCCGTTTTAGCCCACATGGAGGCCACCG
GCGTTCGCTTAGATGTGGCCTATCTGCGCGCCCTGAGCCTGGAAGTTGCA
GAAGAAATCGCCCGTCTGGAGGCAGAGGTGTTTCGTTTAGCCGGCCATCC
GTTCAACCTGAATAGCCGCGACCAGCTGGAACGCGTGCTGTTTGATGAAC
TGGGCTTACCGGCCATTGGCAAGACCGAGAAGACCGGTAAACGTAGTAC
CAGTGCAGCCGTTCTGGAAGCACTGCGTGAAGCCCACCCTATTGTGGAAA
AGATTCTGCAATATCGCGAGTTAACCAAACTGAAGAGCACCTACATCGAT
CCTCTGCCTGATCTGATTCACCCGCGCACCGGTCGTCTGCACACCCGCTTT
AATCAAACAGCCACAGCCACCGGTCGTCTGAGCAGCAGCGATCCGAACTT
ACAGAACATTCCGGTGCGCACCCCTCTGGGCCAACGCATTCGTCGCGCCT
TCATTGCCGAAGAAGGCTGGTTACTGGTGGCCCTGGACTACAGCCAGATT
GAGCTGCGTGTTTTAGCCCATCTGAGCGGCGACGAAAACCTGATTCGCGT
GTTTCAAGAGGGCCGCGATATTCATACAGAGACCGCCAGCTGGATGTTTG
GTGTTCCTCGCGAAGCCGTTGATCCGCTGATGCGCCGCGCAGCCAAAACA
ATTAACTTCGGTGTGCTGTATGGTATGAGCGCCCACCGTTTAAGCCAGGA
GCTGGCCATTCCGTATGAAGAGGCCCAGGCATTTATCGAGCGCTACTTCC
AGAGTTTCCCTAAGGTGCGTGCATGGATCGAGAAAACACTGGAGGAAGG
TCGCCGTCGTGGCTACGTGGAAACCCTGTTTGGCCGCCGTCGCTATGTGC
CGGATCTGGAAGCCCGCGTGAAAAGCGTTCGTGAGGCAGCAGAGCGCAT

GGCATTTAATATGCCGGTGCAGGGTACCGCAGCCGACCTGATGAAACTGG
CAATGGTGAAGCTGTTTCCGCGTCTGGAAGAGATGGGCGCCCGCATGTTA
CTGCAGGTGCACGACGAACTGGTTCTGGAGGCACCGAAAGAACGTGCCG
AAGCAGTGGCACGCCTGGCCAAAGAGGTTATGGAAGGCGTTTATCCGTTA
GCCGTTCCGCTGGAGGTGGAGGTTGGCATTGGCGAAGACTGGCTGAGTGC
AAAAGAATAA

SEQ ID NO:2 (the amino acid sequence of wild-type Taq enzyme is as follows)

In some preferred embodiments, wherein the mutants of Taq enzyme comprise an amino acid sequence having at least 80% identity with the amino acid sequence shown in SEQ ID NO:1; more preferably, the mutants of Taq enzyme comprise an amino acid sequence having at least 90% identity with the amino acid sequence shown in SEQ ID NO:1; more preferably, the mutants of Taq enzyme comprise an amino acid sequence having at least 95% identity with the amino acid sequence shown in SEQ ID NO:1. sequence shown in SEQ ID NO:1. More preferably, the mutants of Taq enzyme comprise an amino acid sequence having at least 95% identity with the amino acid sequence shown in SEQ ID NO:1.

Some preferred embodiments of the present invention provide a nucleotide molecule, wherein the nucleotide molecule encoding a mutant of Taq enzyme described in the first aspect of the present invention.

Some preferred embodiments of the present invention provide a vector comprising the nucleic acid molecule described in the second aspect of the present invention.

Some preferred embodiments of the present invention provide a host cell, wherein the host cell containing a nucleotide molecule described in the second aspect of the present invention, or a chromosome integrating a nucleotide molecule described in the second aspect of the present invention.

In some preferred embodiments, wherein the host cell is a prokaryotic cell, or a eukaryotic cell.

In some preferred embodiments, wherein the prokaryotic cell is E. coli.

In some preferred embodiments, wherein the eukaryotic cell is a yeast cell.

Some preferred embodiments of the present invention provide a kit containing mutants of Taq enzyme described in the first aspect of the present invention.

Some preferred embodiments of the present invention provide a method of preparing the mutants of Taq enzyme described in the first aspect of the present invention, wherein the method comprising the steps of.
(i) culturing the host cell described in the fourth aspect of the present invention under suitable conditions so as to express the mutants of Taq enzyme; and
(ii) isolating the mutants of Taq enzyme.

Some preferred embodiments of the present invention provide a use for the kit described in the fifth aspect of the present invention which is used for DNA sequencing, DNA labeling, primer extension, amplification, and the like.

As used herein, the term "amino acid" in its broadest sense refers to any compound and/or substance that can be incorporated into a polypeptide chain. In some embodiments, the amino acid has the generic structure H2N-C(H)(R)-COOH. In some embodiments, the amino acid is a naturally occurring amino acid. In some embodiments, the amino acid is a synthetic amino acid; in some embodiments, the amino acid is a D-amino acid; in some embodiments, the amino acid is an L-amino acid." A "standard amino acid" refers to any of the twenty standard L-amino acids typically found in naturally occurring peptide. A "non-standard amino acid" is any amino acid other than a standard amino acid, whether prepared synthetically or obtained from natural sources.

As used herein, "synthetic amino acid" includes chemically modified amino acids, including, but not limited to, salts, amino acid derivatives (e.g., amides) and/or substituents. Amino acids, including carboxyl and/or amino-terminal amino acids in peptide, can be modified by methylation, amidation, acetylation, and/or substitution with other chemicals without adversely affecting their activity. The amino acids may have disulfide bonds. The term "amino acid" is used interchangeably with "amino acid residue" and may refer to free amino acids and/or amino acid residues of the peptide. Whether the term refers to free amino acids or peptide residues will be apparent from the context in which the term is used. It should be noted that all amino acid residue sequences are represented herein by the left-right orientation of the formula in the conventional direction of amino terminus to carboxy terminus.

As used herein, the term "mutation" refers to the introduction of a change in the parental sequence, including, but not limited to, substitutions, insertions, deletions (including truncations). Consequences of a mutation include, but are not limited to, the creation of a new characteristic, property, function, phenotype or trait not found in the protein encoded by the parental sequence. The term "mutant" refers to a modified protein, wherein the modified protein exhibits altered characteristics when compared to the parental protein.

As used herein, the term "% homology" is used herein interchangeably with the term "% identity" and refers to the identity level of nucleic acid or amino acid sequence between nucleic acid sequences encoding any of the polypeptides of the invention or amino acid sequences of the polypeptides of the invention when compared using a sequence comparison program.

As used herein, the term "nucleotide" refers to a monomeric unit of DNA or RNA consisting of a sugar portion (pentose sugar), a phosphate ester, and a nitrogenous heterocyclic base.

The base is attached to the sugar portion via the glycosidic carbon (the 1' carbon of the pentose sugar), and the combination of the base and the sugar is a nucleoside. When a nucleoside comprises a phosphate group bonded to the 3' or 5'-position of the pentose sugar, it is called a nucleotide.

Sequences of operably linked nucleotides are commonly referred to herein as "base sequences" or "nucleotide sequence juxtapositions" and are represented herein in such a form that their left-to-right orientation is in the conventional direction from 5'-terminal to 3'-terminal.

As used herein, the term "vector" refers to a nucleoside construct designed for transfer between different host cells.

The term "expression vector" refers to a vector that is capable of incorporating and expressing a heterologous DNA fragment in a foreign cell.

Many prokaryotic and eukaryotic expression vectors are commercially available. The selection of suitable expression vectors is within the knowledge of those skilled in the art.

As used herein, the term "host cell" refers to a cell that has been invaded by a target gene, wherein the target gene can invade the cell in the form of binding to a vector (e.g., virus, chromosome, or plasmid) to achieve replication.

As used herein, the term "chromosomal integration" refers to integration by homologous recombination of multiple copies of the target gene into a sufficiently defined locus.

### Preparation of mutants of Taq enzyme

The Taq enzyme gene sequences of the present invention can be obtained by conventional methods available to those of ordinary skill in the art, such as total synthesis or PCR synthesis. A preferred synthesis method is asymmetric PCR.

Asymmetric PCR is a method in which a large amount of single stranded DNA (ssDNA) is produced after PCR amplification using an unequal pair of primers. These pairs of primers are called non-restriction primers and restriction primers, and their ratio is generally 50-100: 1.

During the first 10-15 cycles of the PCR reaction, the amplification product is mainly double-stranded DNA, but when the restriction primers (low concentration primers) are depleted, the non-restriction primer (high concentration primers) guided PCR produces a large amount of single-stranded DNA.

Primers for PCR can be appropriately selected based on the sequence information of the present invention disclosed herein and can be synthesized by conventional methods. Amplified DNA/RNA fragments can be separated and purified by conventional methods such as gel electrophoresis.

Mutants of Taq enzyme the present invention can be expressed or produced by conventional recombinant DNA techniques comprising the steps of.
(1) transforming or transducing a suitable host cell with a polynucleotide encoding a protein of the invention, or with a recombinant expression vector containing the polynucleotide.
(2) culturing the host cells in a suitable medium.
(3) isolating and purifying the target protein from the medium or cells to obtain a Taq enzyme mutant.

Methods known to those skilled in the art can be used to construct expression vectors containing DNA sequences encoding the Taq enzyme mutants of the present invention encoding and suitable transcriptional/translational control signals, preferably the commercially available vector: pET28.

These methods include in vitro recombinant DNA techniques, DNA synthesis techniques, in vivo recombination techniques and the like. The DNA sequence can be efficiently attached to the appropriate promoter in the expression vector to direct mRNA synthesis.

The expression vector also includes a ribosome binding site for translation initiation and a transcription terminator. In addition, the expression vector preferably comprises one or more selective marker genes to provide phenotypic traits for selection of host cells for transformation.

The recombinant vector comprises in the 5' to 3' direction: a promoter, a target gene and a terminator.

If desired, the recombinant vector may also include the following elements: a protein purification tag; a 3' polyribonucleotide signal; an untranslated nucleic acid sequence; a transporting and targeting nucleic acid sequence; a selection marker (antibiotic resistance gene, fluorescent protein, etc.); an enhancer; or an operon.

Methods for preparing recombinant vectors are well known to those of ordinary skill in the art. Expression vectors may be a bacterial plasmid, phage, yeast plasmid, plant cell virus, mammalian cell virus, or other vectors. In short, any plasmid and vector can be employed as long as it is capable of replicating and stabilizing in the host.

A person of ordinary skill in the art may employ well known methods for constructing a vector containing a promoter of the present invention and/or a target gene sequences of the present invention.

These methods include in vitro recombinant DNA techniques, DNA synthesis techniques, recombinant DNA in vitro, and the like.

The expression vectors of the present invention, can be used to transform appropriate host cells to cause the host to transcribe a target RNA or express a target protein. The host cell may be a prokaryotic cell, such as *Escherichia coli*, *Corynebacterium glutamicum*, *Brevibacterium flavum*, *Streptomyces* spp. and *Agrobacterium* spp;or a lower eukaryotic cell, such as a yeast cell; or a higher eukaryotic cell, such as a plant cell. One of ordinary skill in the art knows how to select the appropriate vector and host cell. Transformation of host cells with recombinant DNA can be performed by conventional techniques known to those of skill in the art. When the host is a prokaryote (e.g., E. coli), it can be treated with CaCl2. Electroporation can also be used.

When the host is eukaryotic, the following DNA transfection methods are available: calcium phosphate co-precipitation, conventional mechanical methods (e.g., microinjection, electroporation, liposome packaging, etc.).

Transformation ofplants can also be performed using Agrobacterium transformation or gene gun transformation methods, such as leaf disk method, young embryo transformation method, bud immersion method, etc. For transformed plant cells, tissues or organs, theycan be regenerated into plants by conventional methods to obtain transgenic plants.

In order to make the objects, technical solutions and advantages of the embodiments of the present invention clearer, the present invention is further elaborated below in connection with specific embodiments. It should be understood that these embodiments are used only to illustrate the present invention and are not intended to limit the scope of the present invention.

The experimental methods for which specific conditions are not indicated in the following embodiments, and the experimental methods for which detailed conditions are not indicated in the following embodiments, are generally in accordance with conventional conditions such as those described in Sambrook. J et al.(ed.) Molecular Cloning: A Laboratory Manual, by U.S.A. (Translated by Huang Peitang et al., Beijing: Science Press, 2002), or in accordance with the conditions recommended by the manufacturer. Unless otherwise indicated, percentages and parts are weight percentages and weight parts. The experimental materials and reagents used in the following embodiments are available from commercially available sources if not otherwise indicated.

Unless otherwise indicated, technical and scientific terms used herein have the same meanings as are commonly understood by one of ordinary skill in the art to which this application belongs, and it is to be noted that the terms used herein are intended only to describe specific embodiments and is not intended to limit the exemplary embodiments of the present application.

### Example 1: Preparation of Taq enzyme mutant Taq-aCM

Steps for the preparation of Taq enzyme mutants.

Step 1: Taq-aCM mutant plasmid was prepared by using wild-type Taq enzyme expression vector as template and QuikChange Lightning Multi-site Mutagenesis Kit, in which the wild-type expression vector was preserved by Daan Gene Co., Ltd. and the vector was pET2 8a with His6 tag at the N-terminal. The mutant plasmids were sent for sequencing, and the sequencing results indicated that the mutant vectors were successfully constructed.

### Step 2: Recombinant Plasmid Transformation of E. coli BL21(DE3)

Under ice bath conditions, taking 1 µ L plasmid, and added to 30 µ L Escherichia coli competent BL21 (DE3). After placing in an ice bath for 20 minutes, heat shock in the 42 °C water bath for 45 seconds and immediately transfer it to ice for 2 minutes. Add 400 µ L SOC medium without antibiotics, 37 °C, 220 rmp shake culture for 50 minutes. 100 µL of the bacterial solution was evenly spread onto LB plates containing 100 µg/mL kanamycin resistant and incubated overnight at 37°C in an incubator.

### Step 3: Expression of target protein

The monoclones from step 2 were picked and aseptically inoculated into 100 µg/mL kanamycin resistant TB medium, shaken incubated at 37°C, 220 rpm until the OD60 was between 0.6 and 0.8, and induced with IPTG (at a final concentration of 0.1 mM), and then left to incubate at 37 °C and 18 °C, respectively, overnight.The group without IPTG was used as a control. The incubation period was 3 hours at 37 °C. The experiments were repeated once for each group. The samples were ultrasonically broken and analyzed by SDS-PAGE.

### Step 4: Purification of Taq-aCM

Shake flask culture with TB medium for 1.5 L of bacterial solution , and the expression conditions were the same as those for the target proteins in step 3). Collect the bacterium by centrifugation, the wet weight of the two proteins is about 30 g.

Weigh about 4 g of bacterium, add 35 ml of Lysis Buffer and resuspend on ice. After ultrasonication and centrifugation at 20,000 rpm for 30 min at 4°C, the supernatant was extracted and filtered through a 0.22 µm needle filter. t. The supernatant was subjected to affinity chromatography using a Ni-Column, and the linear elution was carried out using 0-60% Buffer B, and the eluate of the eluted main peak was taken and subjected to ion-exchange elution using a HisTrapTM Q-HP Purification Column, and the linear elution was carried out using 0-60% Buffer Cp.

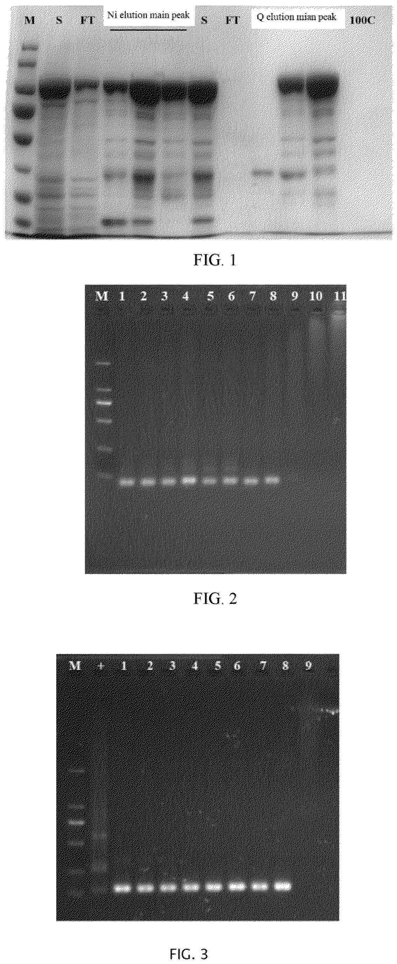

The electropherogram is shown in Figure 1. To calculate the expression content of the target protein, Taq-aCM was 2.5 mg/mL, where the concentration of the solution used is shown follows:
Buffer B: 50mM Tris, 50mM NaCl, 500mM Imidazole, 5% Glycerol, pH8.5;
Lysis Buffer: 50mM Tris,300mM NaCl,5% Glycerol, pH8.5;
Buffer C: 100mM Tris, 1M NaCl, 10% Glycerol, pH8.5.

### Example 2: Test of NaCl resistance of T Taq-aCM

Different NaCl concentration gradient solutions were prepared (NaCl concentration gradient configurations are shown in Table 1 below), Buffer A and Buffer B were prepared (Buffer A and Buffer B formulations are shown in Table 1 below), and the NaCl concentration gradient solutions were formed by mixing Buffer A and Buffer B in different ratios.

**Table 1**

| NaCl concentration /mM | 0 | 10 | 20 | 30 | 40 | 50 | 60 | 70 | 80 | 90 | 100 |
|---|---|---|---|---|---|---|---|---|---|---|---|
| Buffer A/µL | 0 | 1 | 2 | 3 | 4 | 5 | 6 | 7 | 8 | 9 | 10 |
| Buffer B/µL | 10 | 9 | 8 | 7 | 6 | 5 | 4 | 3 | 2 | 1 | 0 |

The resistance of Taq-aCM to NaCl was determined using wild-type Taq enzyme as a positive control.

The PCR reaction system was prepared according to the formulation in Table 2, in which the RV and M4 primers were purchased from TaKaRa, and the 5X Fast Taq Buffer, pUC19 plasmid were kept by Daan Gene Co., Ltd..

The amount of enzyme added was 5 U per 10 µL of PCR reaction system. pUC19 plasmid was used as the template for amplification experiments, and the PCR products were subjected to 2% agarose gel electrophoresis.

**Table 2**

| reagents | BufferA | BufferB |
|---|---|---|
| RV(10pmol) | 1.2µL | 1.2µL |
| M4(10pmol) | 1.2µL | 1.2µL |
| pUC19 plasmid | 6µL | 6µL |
| 5X Fast Taq Buffer | 12µL | 12µL |
| wild type Taq/Taq 2C2/Taq 2C2 Mut | 30U | 30U |
| 1M NaCl | 6µL | 0µL |
| ddH₂O | 31.6µL | 37.6µL |

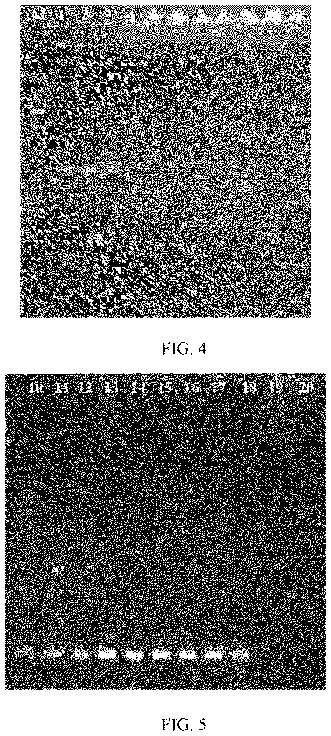

Reaction conditions: 95°C for 2 min, (95°C for 15 s, 44°C for 15 s, 72°C for 1 min) × 30 cycles, 72°C for 1 min, and the electrophoresis results of amplified products are shown in Figures 3 and 4.

As in Figure 2, 1~11 are the PCR product lanes of wild-type Taq enzyme at 0, 10, 20, 30, 30, 40, 50, 60, 70, 80, 90, 100 mM NaCl concentration, respectively;
As in Figure 3, 1~9 are PCR product lanes of Taq-aCM at 110, 120, 130, 140, 150, 160, 170, 180, 190 mM NaCl concentration, respectively.

According to Fig. 2 and Fig. 3, it can be obtained that the resistance of wild-type Taq enzyme to NaCl was 70 mM, and the resistance of Taq-aCM to NaCl reached 180 mM, so the Taq enzyme mutant had a significantly improved NaCl resistance compared with wild-type Taq enzyme.

### Example 3, Taq-aCM anti-KCl performance test

Different KCl concentration gradient solutions (Table 3) were prepared by referring to the method in Example 2, and NaCl was replaced with KCl in BufferA.

**Table 3**

| KCl concentration /mM | 0 | 10 | 20 | 30 | 40 | 50 | 60 | 70 | 80 | 90 | 100 |
|---|---|---|---|---|---|---|---|---|---|---|---|
| Buffer A/µL | 0 | 1 | 2 | 3 | 4 | 5 | 6 | 7 | 8 | 9 | 10 |
| Buffer B/µL | 10 | 9 | 8 | 7 | 6 | 5 | 4 | 3 | 2 | 1 | 0 |

Taq-aCM resistance to KCl was tested according to the same method as in Example 2, and the electrophoresis results of the amplified products are shown in FIGS. 4 and 5.

As in FIG. 4, 1~11 are the wild-type Taq enzyme PCR product lanes at KCl concentrations of 50, 80, 100, 150, 160, 170, 180, 190, 200, 250, and 300 mM, respectively;
As in FIG.5, 10~20 are PCR product lanes of Taq-aCM at KCl concentrations of 50, 80, 100, 150, 160, 170, 180, 190, 200, 250, and 300 mM, respectively; + is a positive control.

According to Figs. 4 and 5, the resistance of wild-type Taq enzyme to KCl was 100 mM, and the Taq mutant Taq-aCM reached resistance to NaCl of 200 mM; thus, the Taq enzyme mutant showed significantly improved resistance to KCl as compared with the wild-type Taq enzyme.

### Example 4, Taq-aCM anti-EDTA whole blood performance test

Different whole blood concentration gradient solutions (Table 4) were prepared by referring to the method in Example 2, wherein NaCl in BufferA was replaced with EDTA whole blood. The volume fraction was calculated according to the volume of blood added to the PCR reaction system.

**Table 4**

| EDTA whole blood concentration/ (V/V) | 0 | 10 | 20 | 30 | 40 | 50 | 60 | 70 | 80 | 90 | 100 |
|---|---|---|---|---|---|---|---|---|---|---|---|
| Buffer A/µL | 0 | 1 | 2 | 3 | 4 | 5 | 6 | 7 | 8 | 9 | 10 |
| Buffer B/µL | 10 | 9 | 8 | 7 | 6 | 5 | 4 | 3 | 2 | 1 | 0 |

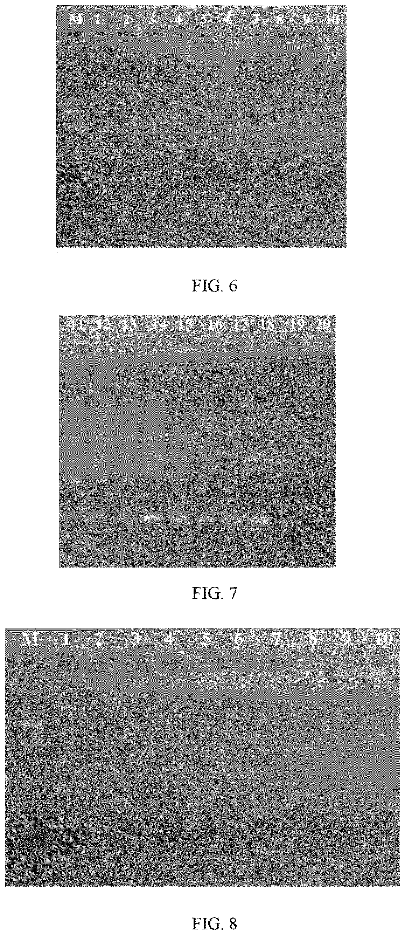

Taq-aCM was tested for resistance to EDTA whole blood according to the same method as in Example 2, and the results of electrophoresis of the amplified products are shown in FIG. 6 and FIG. 7.

As in FIG. 6, 1~10 are the PCR product lanes of wild-type Taq enzyme at EDTA blood concentration of 5, 10, 15, 20, 25, 30, 35, 40, 45, and 50% (V/V), respectively;
As in FIG.7, 11~20 are the PCR product lanes of Taq-aCM at EDTA blood concentration of 5, 10, 15, 20, 25, 30, 35, 40, 45, 50% (V/V), respectively.

According to Figures 6 and 7, it can be obtained that the resistance of wild-type Taq enzyme to EDTA whole blood was 5% (volume fraction was calculated based on the volume of blood added to PCR as a percentage of the total volume of the PCR system), and Taq mutant enzyme, Taq-aCM, was 45% resistant to EDTA whole blood, so the mutant of Taq enzyme has significantly improved its resistance to EDTA whole blood in comparison with wild-type Taq enzyme.

### Example 5: Taq-aCM anti-heparin whole blood performance test

Different whole blood concentration gradient solutions (Table 5) were prepared by referring to the method in Example 2, wherein NaCl in BufferA was replaced with heparin whole blood, and calculating the volume fraction according to the volume of blood added to the PCR reaction system.

**Table 5**

| | | | | | | | | | | | |
|---|---|---|---|---|---|---|---|---|---|---|---|
| Heparin whole blood concentration | 0 | 10 | 20 | 30 | 40 | 50 | 60 | 70 | 80 | 90 | 100 |

| / (V/V) | | | | | | | | | | | |
|---|---|---|---|---|---|---|---|---|---|---|---|
| Buffer A/µL | 0 | 1 | 2 | 3 | 4 | 5 | 6 | 7 | 8 | 9 | 10 |
| Buffer B/µL | 10 | 9 | 8 | 7 | 6 | 5 | 4 | 3 | 2 | 1 | 0 |

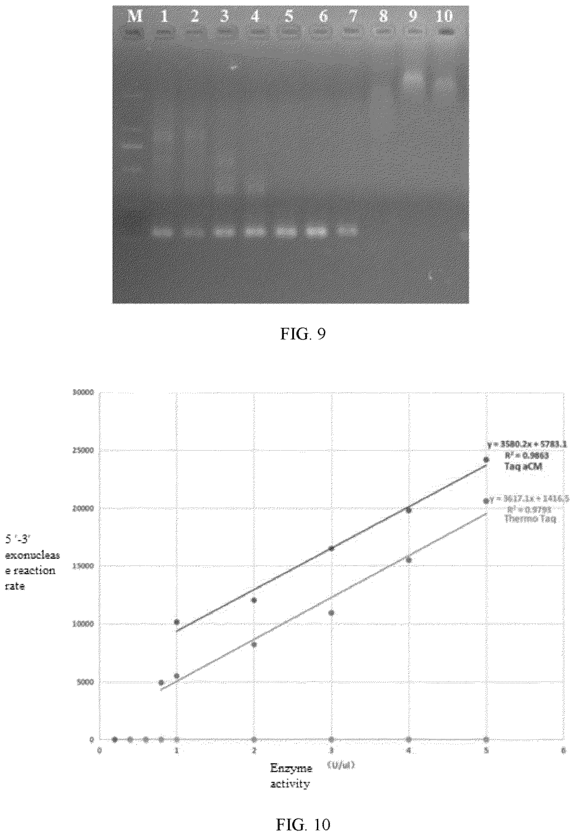

Taq-aCM was tested for resistance to heparin whole blood according to the same method as in Example 2, and electrophoresis results of the amplified products are shown in FIG. 8 and FIG. 9.

As in FIG. 8, 1~10 are the PCR product lanes of wild-type Taq enzyme at heparin blood concentration of 5, 10, 15, 20, 25, 30, 35, 40, 45, and 50% (V/V), respectively;
As in FIG.9, 1 to 10 are the PCR product lanes of Taq-aCM at heparin blood concentration of 5, 10, 15, 20, 25, 30, 35, 40, 45, and 50% (V/V), respectively;

According to Figures 8 and 9, it can be obtained that the wild-type Taq enzyme has 0% resistance to heparin whole blood, and the Taq mutant enzyme Taq-aCM has 35% resistance to heparin whole blood, so that the Taq enzyme mutant has a significantly higher resistance to heparin whole blood than the wild-type Taq enzyme.

### Example 6: Taq-aCM 5'~3' exonuclease activity test

5'~3' exonuclease activity was assayed by fluorescent probe PCR with wild-type Taq enzyme (ThermoFisher Scientific) as a positive control, diluted to 1U, 2U, 3U, 4U, 5U, and Taq-aCM was similarly diluted to the same activity concentration.

The reaction system was prepared as shown in Table 5 below (where 10X Taq Buffer was purchased from TaRaKa):

**Table 5**

| Reagent | mixing volume |
|---|---|
| 10× Taq Buffer | 2.5ul |
| 25mM MgCl2 | 4.5ul |
| 2.5mM dNTPs | 0.2ul |
| Probe2(10pmol) | 0.5ul |
| DNA polymerase test 2(10pmol) | 0.25µl |
| ddH2O | 16.05µL |

PCR reaction conditions: 10 minutes at 95°C, (95°C for 10 seconds, 55°C for 30 seconds, read fluorescence) × 40 cycles. Using the reaction cycle as the X-axis and the fluorescence value (RFU) corresponding to each cycle as the Y-axis, an RFU-cycling graph was made.

The known enzyme concentration was used as the X-axis, and the initial slope corresponding to the RFU-cycling graph was used as the Y-axis to fit the standard curve, and the slope of the linear regression equation obtained could indicate the 5'~3' exonuclease activity. The results are shown in Fig. 10.

According to Fig. 10, the 5'~3' exonuclease activity of Taq-aCM after mutation was basically the same as that of wild-type Taq enzyme.

The Taq enzyme mutant Taq2C2 was prepared by the method in reference *Mutant Taq DNA polymerases with improved elongation ability as a useful reagent for genetic engineering. Front Microbiol 5:461. doi: 10.3389*/*fmicb.2014.00461* and tested for its 5'~3' exonuclease activity under the same conditions, and the Taq-2C2 5'~3' exonuclease activity was 66% of that of the wild-type Taq enzyme.

It will be understood by one of ordinary skill in the art that the above embodiments are specific embodiments for realizing the present invention, and that various changes can be made thereto in form and detail in practical application without departing from the spirit and scope of the present invention.

## Claims

1. A mutant of Taq enzyme, wherein the mutant of Taq enzyme comprising: an amino acid sequence having at least 70% identity to the amino acid sequence shown in SEQ ID NO:2, wherein the amino acid sequence being mutated at one or more sites selected from the group consisting of: D335, G499, E634, F769, and a combination thereof.

2. The mutant of Taq enzyme according to claim 1, wherein the amino acid sequence is mutated at one or more sites selected from the group consisting of: D335V, G499K, E634G and F769I.

3. The mutant of Taq enzyme according to claim 2, wherein the amino acid sequence is mutated at four sites in the following group: D335V, G499K, E634G and F769I.

4. The mutant of Taq enzyme according to any one of claims 1 to 3, wherein the mutant of Taq enzyme has a resistance to sodium chloride of not less than 70 mM;
and/or, wherein the mutant of Taq enzyme is resistant to potassium chloride not less than 100 mM.

5. The mutant of Taq enzyme according to any one of claims 1 to 3, wherein the mutant of Taq enzyme is resistant to EDTA whole blood by not less than 5%;
and/or, wherein the mutant of Taq enzyme is resistant to heparin whole blood by not less than 1%.

6. A nucleotide molecule, wherein the nucleotide molecule encoding a mutant of Taq enzyme in any one of claims 1 to 5.

7. A vector, wherein the vector containing the nucleic acid molecule in claim 6.

8. A host cell, wherein the containing a nucleotide molecule in claim 7, or the chromosome integrating a nucleotide molecule in claim 5.

9. A kit, wherein the kit containing mutants of Taq enzyme in any one of claims 1 to 5.

10. A method of preparing the mutants of Taq enzyme in claim 1, comprising the steps of.
(i) culturing the host cell in claim 8 under suitable conditions so as to express the mutants of Taq enzyme; and
(ii) isolating the mutants of Taq enzyme.
